# EUROPEAN PATENT APPLICATION

(11) **EP 4 413 853 A1**
(43) Date of publication of application: **14.08.2024**
(21) Application number: 22878132.4
(22) Date of filing: 07.03.2022
(51) Int. Cl.: A01H 1/02, G06Q 50/02, A01G 7/00

(54) **AGRICULTURAL ASSISTANCE SYSTEM, AGRICULTURAL ASSITANCE DEVICE, AGRICULTURAL ASSISTANCE METHOD, AND AGRICULTURAL ASSISTANCE PROGRAM**

(30) Priority: 05.10.2021 JP 2021164227
(71) Applicant: Harvestx Inc., Tokyo, 113-0033 (JP)
(72) Inventor: ICHIKAWA, Yuki, Tokyo 113-0033 (JP); WATANABE, Aoi, Tokyo 113-0033 (JP)
(74) Representative: AWA Sweden AB
(86) International application number: PCT/JP2022/009643
(87) International publication number: WO 2023/058259

(57) **Abstract**

An agricultural assistance system includes: an operation mechanism that contacts a plant to perform a predetermined operation; and a control unit. The control unit includes: a model generation unit that generates a three-dimensional model of a plant based on a combination of predetermined generation conditions; a training unit that generates a first trained model trained with training data, the training data including: a value of a predetermined parameter in the three-dimensional model; and an image of a plant; an image obtainment unit that obtains an image of a target plant to be evaluated; a model obtainment unit that obtains the first trained model; a parameter estimation unit that estimates, based on the first trained model and the image of the target plant to be evaluated, a value of the predetermined parameter for a predetermined site in the target plant to be evaluated; and an operation instruction unit that causes the operation mechanism to perform the predetermined operation based on the estimated value of the predetermined parameter.

## Description

### [TECHNICAL FIELD]

The present disclosure relates to an agricultural assistance system, an agricultural assistance apparatus, an agricultural assistance method, and an agricultural assistance program.

### [BACKGROUND ART]

In agriculture, techniques of automating pollination of plants such as fruits have been known.

Patent Literature 1 describes a technique for a drone controller that controls a drone having an application attachment for applying pollen or a fruiting agent to pistils.

### [CITATION LIST]

### [PATENT LITERATURE]

**[PATENT LITERATURE 1]** Japanese Patent Laid-Open No. 2021-45055

### [SUMMARY OF INVENTION]

### [TECHNICAL PROBLEM]

The technique described in Patent Literature 1 involves determining the blooming states of flowers based on flower image data obtained from a drone and applying pollen or a fruiting agent to the flowers.

Unfortunately, the technique in Patent Literature 1 may fail to appropriately apply pollen or a fruiting agent to flowers oriented in revolving directions.

There is a need for a technique that enables more appropriately performing agricultural tasks such as pollination.

### [SOLUTION TO PROBLEM]

According to an embodiment, an agricultural assistance system is provided that includes: an operation mechanism that contacts a plant to perform a predetermined operation; and a control unit. The control unit includes: a model generation unit that generates a three-dimensional model of a plant based on a combination of predetermined generation conditions; a training unit that generates a first trained model trained with training data, the training data including: a value of a predetermined parameter in the three-dimensional model; and an image of a plant; an image obtainment unit that obtains an image of a target plant to be evaluated; a model obtainment unit that obtains the first trained model; a parameter estimation unit that estimates, based on the first trained model and the image of the target plant to be evaluated, a value of the predetermined parameter for a predetermined site in the target plant to be evaluated; and an operation instruction unit that causes the operation mechanism to perform the predetermined operation based on the estimated value of the predetermined parameter.

### [ADVANTAGEOUS EFFECT OF INVENTION]

According to the present disclosure, agricultural tasks such as pollination can be performed more appropriately.

### [BRIEF DESCRIPTION OF DRAWINGS]

[Figure 1] Figure 1 is a block diagram illustrating the overall configuration of a system 1.
[Figure 2] Figure 2 is a diagram illustrating the functional configuration of a terminal device 10.
[Figure 3] Figure 3 is a diagram illustrating the functional configuration of a server 20.
[Figure 4] Figure 4 is a diagram illustrating the functional configuration of an agricultural assistance apparatus 30.
[Figure 5] Figure 5 is a diagram illustrating an exemplary external view (perspective view) of the agricultural assistance apparatus 30 according to a first embodiment.
[Figure 6] Figure 6 is a diagram illustrating an exemplary external view (side view) of the agricultural assistance apparatus 30 according to the first embodiment.
[Figure 7] Figure 7 is a diagram illustrating a detailed structure of a main unit 502 of the agricultural assistance apparatus 30.
[Figure 8] Figure 8 is a flowchart illustrating a process in which the agricultural assistance apparatus 30 performs a predetermined operation based on a plant image and a trained model obtained.
[Figure 9] Figure 9 is a diagram illustrating actions through which an operation mechanism 302 of the agricultural assistance apparatus 30 performs a predetermined operation for a predetermined site in a plant based on the value of a predetermined parameter.
[Figure 10] Figure 10 illustrates an exemplary screen that displays notifications to a user managing a cultivated field when tendencies to unhealthiness of plants are detected by the operation mechanism 302 of the agricultural assistance apparatus 30.

### [DESCRIPTION OF EMBODIMENT]

An embodiment of the present disclosure will be described below with reference to the drawings. In the following description, like elements are labeled with like symbols and have like names and functions. Such elements will thus not be repeatedly described in detail.

### <First embodiment>

### <Overview>

The embodiment below describes a technique that involves: obtaining images of plants; estimating, based on a trained model, the values of a predetermined parameter for the plants in a three-dimensional space; and operating, based on the parameter values, an operation mechanism provided at the tip of an arm of an agricultural assistance apparatus.

As an example for comparison with the embodiment, the following configuration will be described. This comparative example is assumed to be a technique of obtaining images of plants, identifying the positions of the pistils of flowers, and automatically pollinating the flowers with an apparatus. For example, a trained model that associates training images of flowers with blooming states is used to determine the blooming states of the flowers in the captured images. According to the orientations of flowers determined to be in bloom, an attachment is driven to pollinate the flowers. In this comparative example, however, the flower orientations are merely orientations on a plane based on the captured images (a two-dimensional plane). Flowers facing along the depth of the image may fail to undergo appropriate blooming determination and pollination, resulting in incomplete pollination with some flowers left unpollinated. This may lead to a reduced crop yield.

To address this, a system 1 to be described in the embodiment provides an agricultural assistance system including: an operation mechanism that contacts a plant to perform a predetermined operation; and a control unit. The control unit includes: a model generation unit that generates a three-dimensional model of a plant based on a combination of predetermined generation conditions; a training unit that generates a first trained model trained with training data, the training data including: a value of a predetermined parameter in the three-dimensional model; and an image of a plant; an image obtainment unit that obtains an image of a target plant to be evaluated; a model obtainment unit that obtains the first trained model; a parameter estimation unit that estimates, based on the first trained model and the image of the target plant to be evaluated, a value of the predetermined parameter for a predetermined site in the target plant to be evaluated; and an operation instruction unit that causes the operation mechanism to perform the predetermined operation based on the estimated value of the predetermined parameter.

Thus, the system 1 provides a technique that enables more appropriately performing agricultural tasks such as pollination.

The system 1 may be used at, for example, scenes where tasks such as pollination, harvesting, and removing leaves are automatically performed in a plant factory, a greenhouse, or a polytunnel. This enables more appropriately performing agricultural tasks such as pollination.

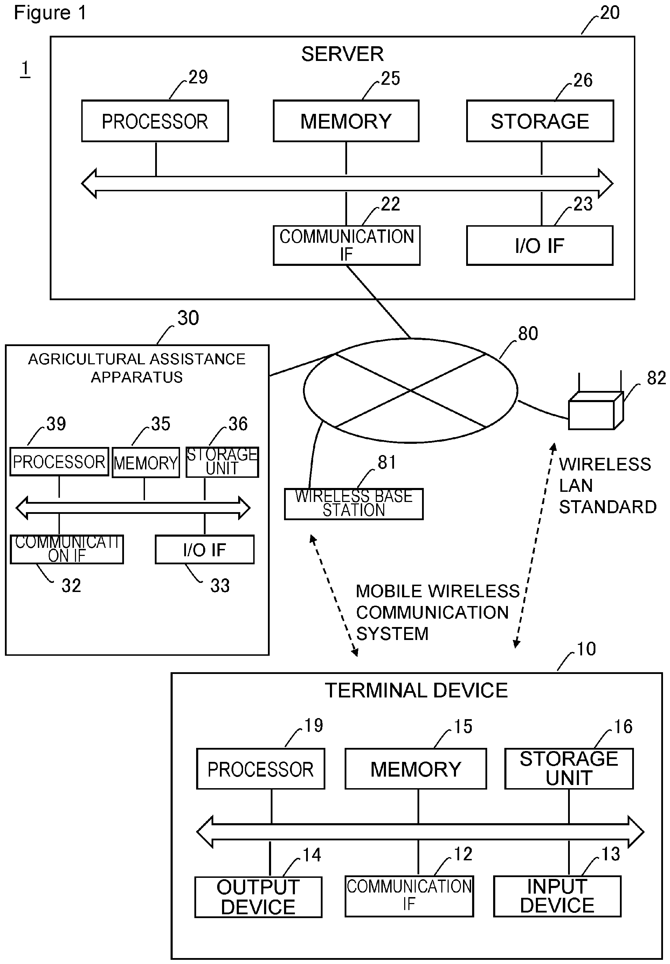

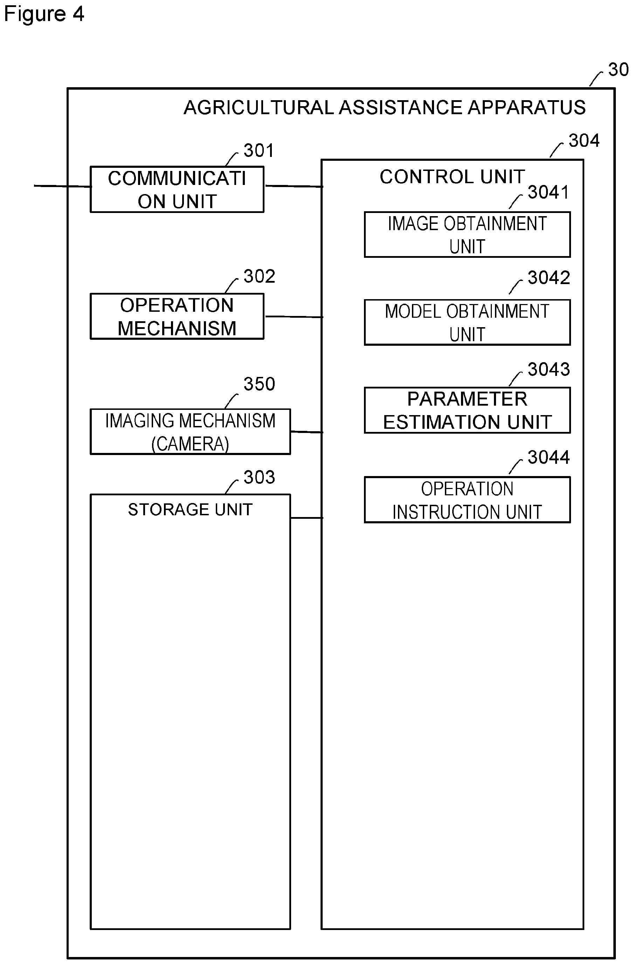

Now, with reference to Figures 1 to 4, configurations of the system 1 according to a first embodiment of the present disclosure will be described. The system 1 is a system for planned cultivation of plants, typically in plant factories, greenhouses, or polytunnels, and may include the following apparatuses.
- an apparatus for automatically pollinating flowers of plants
- an apparatus for automatically harvesting fruits of plants
- an apparatus for automatically removing, pruning, or thinning out leaves or stems of plants
- an apparatus for automatically spreading fertilizer for plants

Specifically, the pollination apparatus may automatically pollinate self-pollinating flowers in which their stigmas receive pollen from flowers of the same plant, as well as automatically pollinate cross-pollinating flowers in which their stigmas receive pollen from flowers of other plants of the same species.

### <1 General view of configuration of system>

Figure 1 illustrates the overall configuration of the system 1 in the first embodiment.

As illustrated in Figure 1, the system 1 includes a terminal device 10 (which may include multiple terminal devices such as 10A, 10B, and 10C, although Figure 1 shows only the terminal device 10), a server 20, and an agricultural assistance apparatus 30. The terminal device 10, the server 20, and the agricultural assistance apparatus 30 are communicatively interconnected via a network 80. The network 80 may include a wired or wireless network.

The terminal device 10 is operated by a user. The terminal device 10 may be implemented by a desktop or laptop personal computer (PC). The terminal device 10 may also be a mobile terminal, for example a tablet computer or a smartphone, that supports a mobile communication system. As illustrated in Figure 1, the terminal device 10 includes a communication interface (IF) 12, an input device 13, an output device 14, a memory 15, a storage unit 16, and a processor 19. The server 20 includes a communication IF 22, an input/output IF 23, a memory 25, a storage 26, and a processor 29. The agricultural assistance apparatus 30 includes a communication IF 32, an input/output IF 33, a memory 35, a storage unit 36, and a processor 39.

The terminal device 10 is communicatively connected with the server 20 and the agricultural assistance apparatus 30 via the network 80. The terminal device 10 is connected to the network 80 by communicating with communication apparatuses, such as a wireless base station 81 that supports communication standards such as 5G and Long Term Evolution (LTE), and a wireless local area network (LAN) router 82 that supports wireless LAN standards such as IEEE (Institute of Electrical and Electronics Engineers) 802.11.

The communication IF 12 is an interface for the terminal device 10 to output and receive signals to communicate with external apparatuses. The input device 13 includes input devices for receiving input operations from the user (e.g., pointing devices such as a touch panel, a touch pad, and a mouse, and a keyboard). The output device 14 includes output devices for presenting information to the user (such as a display and a speaker). The memory 15 is used for temporarily storing programs, as well as items such as data processed by the programs, and may be volatile memory such as dynamic random access memory (DRAM), for example. The storage unit 16 is a storage device for saving data, and may be flash memory or a hard disk drive (HDD), for example. The processor 19 is hardware for executing instruction sets written in programs, and includes components such as arithmetic units, registers, and peripheral circuits.

The server 20 manages information related to trained models to be obtained by the agricultural assistance apparatus. The details of the trained models will be described later.

In an aspect, the server 20 may manage information on a user operating a cultivated field. Specifically, for example, the server 20 may manage the following items as the information on the user operating the cultivated field.
- vegetables and fruits grown by the user in the cultivated field
- the area of the cultivated field managed by the user
- tools and machines possessed by the user
- the gradient of the cultivated field managed by the user
- properties of the soil of the cultivated field managed by the user
- fertilizer used by the user in the cultivated field
- the region where the cultivated field managed by the user is located
- climate information on the region where the cultivated field managed by the user is located (such as average temperatures, rainfalls, and sunshine hours)

The communication IF 22 is an interface for the server 20 to output and receive signals to communicate with external apparatuses. The input/output IF 23 functions as an interface with input devices for receiving input operations from users, and with output devices for presenting information to users. The memory 25 is used for temporarily storing programs, as well as items such as data processed by the programs, and may be volatile memory such as dynamic random access memory (DRAM), for example. The storage 26 is a storage device for saving data, and may be flash memory or a hard disc drive (HDD), for example. The processor 29 is hardware for executing instruction sets written in programs, and includes components such as arithmetic units, registers, and peripheral circuits.

In the embodiment, the above apparatuses such as the terminal device and the server may be collectively regarded as an information processing apparatus. That is, the aggregation of the above apparatuses may be regarded as a single "information processing apparatus," so that the system 1 may be formed as an aggregation of multiple apparatuses. How the functions required for implementing the system 1 according to the embodiment are distributed among one or more hardware items may be determined as appropriate in view of the processing capability of each hardware item and/or specifications required for the system 1.

The agricultural assistance apparatus 30 performs predetermined tasks in the cultivated field based on instructions from a user or on preset conditions. Specifically, for example, the predetermined tasks include the following tasks in the cultivated field managed by the user.
- pollination (applying pollen to pistils)
- harvesting
- removing leaves (removing overlapping leaves and excess leaves)
- spreading fertilizer
- thinning out for removing diseased fruits or leaves

The communication IF 32 is an interface for the agricultural assistance apparatus 30 to output and receive signals to communicate with external apparatuses. The input/output IF 33 functions as an interface with input devices for receiving input operations from the user, and with output devices for presenting information to the user. The memory 35 is used for temporarily storing programs, as well as items such as data processed by the programs, and may be volatile memory such as dynamic random access memory (DRAM), for example. The storage unit 36 is a storage device for saving data, and may be flash memory or a hard disc drive (HDD), for example. The processor 39 is hardware for executing instruction sets written in programs, and includes components such as arithmetic units, registers, and peripheral circuits.

### <1.1 Configuration of terminal device 10>

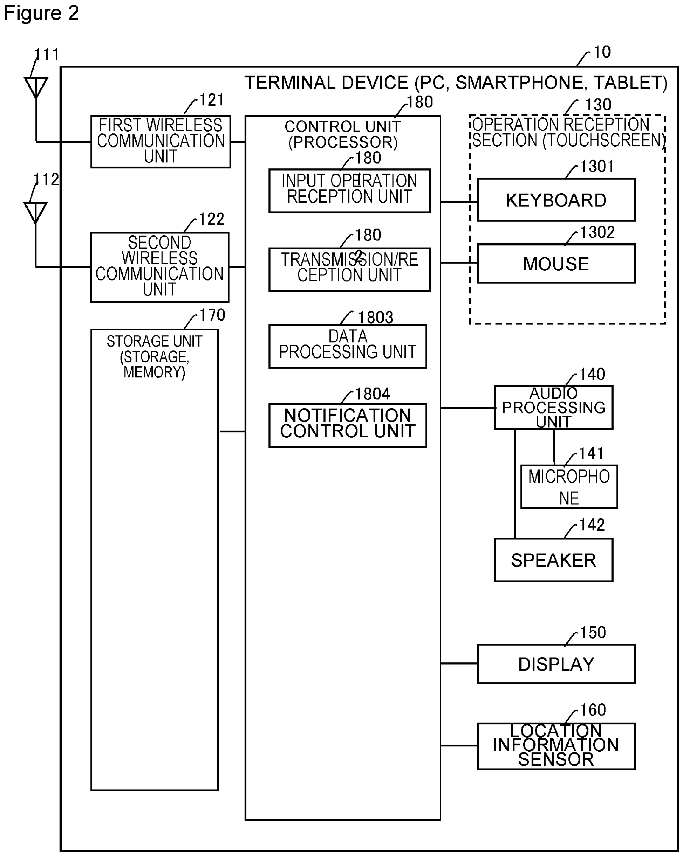

Figure 2 is a block diagram illustrating the functional configuration of the terminal device 10 included in the system 1 in the first embodiment. As illustrated in Figure 2, the terminal device 10 includes antennas (an antenna 111 and an antenna 112), wireless communication units (a first wireless communication unit 121 and a second wireless communication unit 122) corresponding to the respective antennas, an operation reception section 130 (including a keyboard 1301 and a mouse 1302), an audio processing unit 140, a microphone 141, a speaker 142, a display 150, a location information sensor 160, a storage unit 170, and a control unit 180. The terminal device 10 also includes functions and components not specifically shown in Figure 2 (e.g., a battery for holding power, and a power supply circuit that controls power supply from the battery to circuits). As illustrated in Figure 2, the blocks in the terminal device 10 are electrically interconnected by means such as buses.

The antenna 111 emits, as radio waves, signals to be transmitted by the terminal device 10. The antenna 111 also receives radio waves through the air and provides the received signals to the first wireless communication unit 121.

The antenna 112 emits, as radio waves, signals to be transmitted by the terminal device 10. The antenna 112 also receives radio waves through the air and provides the received signals to the second wireless communication unit 122.

The first wireless communication unit 121 performs processing such as modulation and demodulation for the terminal device 10 to transmit and receive signals via the antenna 111 to communicate with other wireless devices. The second wireless communication unit 122 performs processing such as modulation and demodulation for the terminal device 10 to transmit and receive signals via the antenna 112 to communicate with other wireless devices. The first wireless communication unit 121 and the second wireless communication unit 122 are each a communication module that includes components such as a tuner, a received signal strength indicator (RSSI) calculation circuit, a cyclic redundancy check (CRC) calculation circuit, and a radio frequency circuit. The first wireless communication unit 121 and the second wireless communication unit 122 perform modulation and demodulation and frequency conversion for radio signals transmitted and received by the terminal device 10, and provide received signals to the control unit 180.

The operation reception section 130 includes mechanisms for receiving the user's input operations. Specifically, the operation reception section 130 includes the keyboard 1301 and the mouse 1302. The operation reception section 130 may also be configured as a touchscreen that uses a capacitive touch panel, for example, to detect positions touched by the user on the touch panel.

The keyboard 1301 receives input operations by the user of the terminal device 10. The keyboard 1301, which is a device for inputting text, outputs input text information as input signals to the control unit 180.

The mouse 1302 receives input operations by the user of the terminal device 10. The mouse 1302 is a pointing device for operations, such as selection, on objects displayed on the display 150, and outputs, as input signals to the control unit 180, information on positions selected on the screen and information indicating the depression of a button.

The audio processing unit 140 modulates and demodulates audio signals. The audio processing unit 140 modulates signals received from the microphone 141 and provides the modulated signals to the control unit 180. The audio processing unit 140 also provides audio signals to the speaker 142. The audio processing unit 140 may be implemented by a processor for audio processing, for example. The microphone 141 receives audio input and provides audio signals corresponding to the audio input to the audio processing unit 140. The speaker 142 converts audio signals received from the audio processing unit 140 into sound and outputs the sound to the outside of the terminal device 10.

The display 150 displays data, such as images, videos, and text, according to control by the control unit 180. The display 150 may be implemented by a liquid crystal display (LCD) or an organic electro-luminescence (EL) display, for example.

The location information sensor 160 detects the location of the terminal device 10 and may be a global positioning system (GPS) module, for example. A GPS module is a receiver used in a satellite positioning system. In a satellite positioning system, the GPS module in the terminal device 10 receives signals from at least three or four satellites and, based on the signals received, detects the current location of the terminal device 10. For example, if the system 1 permits the user's location information to be referred to, the user's location information may be used to identify information on the location of the cultivated field managed by the user, thereby identifying climate conditions in the cultivated field identified. Further, if the terminal device 10 has information on the user managing the cultivated field, the location information may be used to identify the information on the user managing the cultivated field in the region of interest. The location information sensor 160 may also be a transceiver based on a communication standard used in a short-range communication system between information devices. Specifically, the location information sensor 160 may be a Bluetooth (R) module that uses the 2.4 GHz band to receive beacon signals from other information devices having Bluetooth (R) modules.

The storage unit 170 may be implemented by flash memory, for example, and stores data and programs used by the terminal device 10. In an aspect, the storage unit 170 may store the following items as information on the user managing the cultivated field, for example.
- vegetables and fruits grown by the user in the cultivated field
- the area of the cultivated field managed by the user
- tools and machines possessed by the user
- the gradient of the cultivated field managed by the user
- properties of the soil of the cultivated field managed by the user
- fertilizer used by the user in the cultivated field
- the region where the cultivated field managed by the user is located
- climate information on the region where the cultivated field managed by the user is located (such as average temperatures, rainfalls, and sunshine hours)

The control unit 180 controls the operations of the terminal device 10 by reading programs stored in the storage unit 170 and executing instructions in the programs. The control unit 180 may be an application processor, for example. The control unit 180 operates according to the programs to serve as an input operation reception unit 1801, a transmission/reception unit 1802, a data processing unit 1803, and a notification control unit 1804.

The input operation reception unit 1801 performs the processing of receiving the user's input operations on input devices such as the keyboard 1301. If the user's input operation is received through an input device such as a touch sensitive device (not shown), the input operation reception unit 1801 may identify, based on information on the coordinates at which the user's finger touches on the touch sensitive device, the type of the user's operation among operation types including the following.
- a flick operation
- a tap operation
- a drag (swipe) operation

The transmission/reception unit 1802 performs processing for the terminal device 10 to transmit and receive data to and from external apparatuses, such as the server 20, according to a communication protocol.

The data processing unit 1803 performs the processing of subjecting input data received by the terminal device 10 to arithmetic operations according to programs, and outputting the result of the arithmetic operations to memory or other destinations.

The notification control unit 1804 performs the processing of presenting information to the user. That is, the notification control unit 1804 performs processing such as causing the display 150 to display images and causing the speaker 142 to output sound.

### <1.2 Functional configuration of server 20>

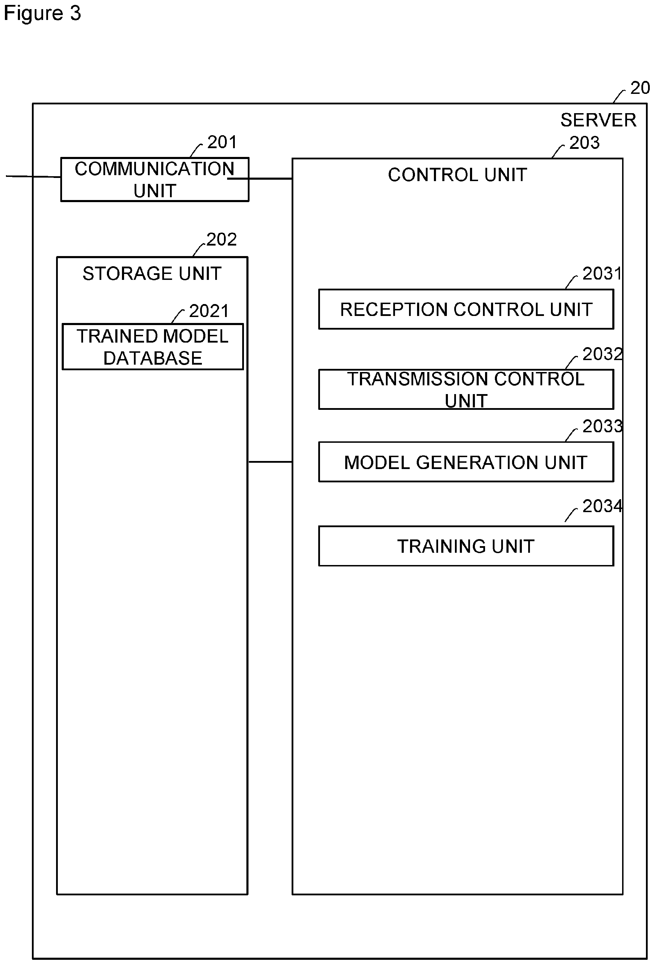

Figure 3 is a diagram illustrating the functional configuration of the server 20. As illustrated in Figure 3, the server 20 serves as a communication unit 201, a storage unit 202, and a control unit 203.

The communication unit 201 performs processing for the server 20 to communicate with external apparatuses.

The storage unit 202 stores data and programs used by the server 20. The storage unit 202 stores a trained model database 2021. In an aspect, the storage unit 202 may store information on the user managing the cultivated field.

The trained model database 2021 is a database for managing information on trained models that associate plant images with the values of predetermined parameters for predetermined sites of plants in three-dimensional models. Not only plant images but also plant videos may be associated with the values of the predetermined parameters.

A reception control unit 2031 controls processing for the server 20 to receive signals from external apparatuses according to a communication protocol.

A transmission control unit 2032 controls processing for the server 20 to transmit signals to external apparatuses according to a communication protocol.

A model generation unit 2033 controls the processing of generating three-dimensional models of plants based on predetermined generation conditions. Specifically, for example, the model generation unit 2033 generates a three-dimensional model of a plant using a combination of elements including the following as the generation conditions for the three-dimensional model.
- a leaf, stem, flower (including a pistil and stamens), or fruit of the plant
- the size of a leaf, stem, flower (including a pistil and stamens), or fruit of the plant
- the number of leaves, stems, flowers (including pistils and stamens), or fruits of the plant
- the color of a leaf, stem, flower (including a pistil and stamens), or fruit of the plant
- information on the background
- information on illumination
- the orientation of a leaf, stem, flower (including a pistil and stamens), or fruit of the plant
- the position of a leaf, stem, flower (including a pistil and stamens), or fruit of the plant

The three-dimensional models may be generated using any existing three-dimensional rendering software.

The server 20 may accumulate, in the storage unit 202, information on the three-dimensional models generated using a combination of the above elements.

A training unit 2034 controls the processing of generating training models that associate the generated three-dimensional models with the values of predetermined parameters for plants, and storing the trained models in the trained model database 2021 in the storage unit 202 of the server 20.

The following illustrates how a trained model is generated in the present disclosure.

For example, the training unit 2034 obtains information on three-dimensional models generated by the model generation unit 2033.

The training unit 2034 then receives, from the user managing the cultivated field, images (or videos) of plants to be associated with the three-dimensional models. The training unit 2034 associates the values of parameters for sites in the plants (the positions, orientations, and positional relationships of pistils) with the values of the parameters for the sites in the three-dimensional models. For example, the training unit 2034 receives an image of a plant in which a pistil is oriented in a predetermined direction. The training unit 2034 then receives, from the user managing the cultivated field, a three-dimensional model in which a pistil is oriented in the same direction as in the image of the plant received, and associates a parameter value in the model with a parameter value in the image.

In response to receiving input of an image of a plant, the server 20 identifies, based on the trained model, the value of a parameter (e.g., orientation or positional relationship) for a predetermined site (e.g., a flower, pistil, or leaf) in the plant and outputs the value as a parameter value for operating the agricultural assistance apparatus. That is, for a pistil oriented in a certain direction (e.g., diagonally backward along the depth), the server 20 outputs information for performing pollination from front of the pistil. The information output here may be information on a normal vector, and the agricultural assistance apparatus may operate the operation mechanism (to be described later) based on the output information on the normal vector.

Thus, the user managing the cultivated field can cause tasks in the cultivated field to be automatically performed in an accurate and fine manner under various conditions.

In an aspect, the server 20 may store, in addition to information on the above-illustrated trained model (a first trained model), information on multiple trained models in the storage unit. Specifically, for example, the server 20 may store the following trained models.
- a trained model (a second trained model) generated by training with: at least one of ventilation and sunlight in the cultivated field; and fruit yields in the harvest season
- a trained model (a third trained model) that associates images of fruits, the weights of the fruits, the sugar contents of the fruits, and the qualities of the fruits with each other
- a trained model (a fourth trained model) that associates images of plants with images of the plants having diseases
- a trained model (a fifth trained model) that associates images of plants with the nutrition states of the plants

The following illustrates how each of the above trained models is generated.

In an aspect, the server 20 may store the trained models for each plant species. For example, the above trained models may be stored for each of the plant species grown in the cultivated field, such as strawberry, tomato, melon, and cucumber. In this case, the server 20 may first identify which plant species is shown in an image obtained and then determine a trained model to be provided.

Thus, for multiple plant species grown in the cultivated field, the user managing the cultivated field can readily change the trained model to be obtained and appropriately perform operations.

How the second trained model is generated will be illustrated. The server 20 receives, from the user managing the cultivated field, information on ventilation in the cultivated field (which may be approximated to the densities of the leaves of plants), or sunlight in the cultivated field (which may be sunshine rates at sites in the plants based on the densities of the leaves of the plants). The server 20 then receives, from the user managing the cultivated field, information on fruit yields of the plants in the harvest season, and associates this information with the ventilation or sunlight information. In response to receiving input of an image of a plant, the server 20 outputs a predicted yield based on the second trained model.

In an aspect, based on the output predicted yield, the agricultural assistance apparatus may estimate the positional relationship among leaves that will maximize the yield and, based on the estimation, perform an operation such as removing leaves.

Thus, based on information such as ventilation among leaves, the user managing the cultivated field can cause adjustments to be made automatically to maximize the yield.

How the third trained model is generated will be illustrated. The server 20 may receive images of fruits and their weights, sugar contents, and qualities from the user managing the cultivated field and associate these items with each other. In response to receiving input of an image of a fruit and its weight and sugar content, the server 20 outputs the quality of the fruit.

Thus, the user managing the cultivated field can quantitatively evaluate the quality of fruits.

How the fourth trained model is generated will be illustrated. The server 20 receives, from the user managing the cultivated field, information on three-dimensional plant models generated based on images of diseased plants. Here, the user managing the cultivated field may generate diseased-plant models in which one or more of the conditions illustrated below are set.
- the sites of a disease
- the degree of a disease (such as the area of the disease on leaves or flowers)
- the site of a disease in an individual plant

The server 20 then receives images of plants that show tendencies to diseases, and associates the images with the three-dimensional models. If part of leaves exhibits a certain disease (such as, but not limited to, anthracnose, phylloxerae, or mildew), the server 20 outputs information on the sites showing the tendency to the disease.

In an aspect, based on the output information on the sites showing the tendency to the disease, the agricultural assistance apparatus may perform an operation such as picking or thinning out the sites.

Thus, for a vast cultivated field, the user managing the cultivated field can immediately detect a tendency to a disease in its early stage and prevent damages that would be caused by the disease.

How the fifth trained model is generated will be illustrated. The server 20 receives, from the user managing the cultivated field, information on three-dimensional plant models generated based on images of plants in different nutrition states (such as undernutrition and overnutrition). The server 20 then receives images of plants that show tendencies to such nutrition states, and associates the images with the three-dimensional models. If some plants in the cultivated field exhibit undernutrition, the server 20 outputs information on the sites showing the tendency to undernutrition.

In an aspect, based on the output information on the sites showing the tendency to undernutrition, the agricultural assistance apparatus may adjust the amount or percentage of fertilizer to be spread.

Thus, the user managing the cultivated field can recognize situations such as unevenly spread fertilizer. This contributes to harvesting uniform-quality crops such as fruits.

The training unit 2034 controls the processing of storing the generated trained models in the trained model database 2021 in the storage unit 202 of the server 20. Specifically, for example, the training unit 2034 may send a trained model to the agricultural assistance apparatus 30 in response to receiving an operation instruction from the user managing the cultivated field. Alternatively, in response to receiving an image of a plant from the agricultural assistance apparatus 30, the training unit 2034 may send a trained model corresponding to the plant.

### <1.3 Functional configuration of agricultural assistance apparatus 30>

Figure 3 is a diagram illustrating the functional configuration of the agricultural assistance apparatus 30. As illustrated in Figure 3, the agricultural assistance apparatus 30 includes a communication unit 301, an operation mechanism 302, a storage unit 303, a control unit 304, and an imaging mechanism 350.

The communication unit 201 enables the agricultural assistance apparatus 30 to communicate with external apparatuses.

The operation mechanism 302 enables the agricultural assistance apparatus 30 to perform predetermined operations for plants. The predetermined operations may include operations for the following tasks, for example.
- pollination (applying pollen to pistils)
- harvesting
- removing leaves (removing overlapping leaves and excess leaves)
- spreading fertilizer
- thinning out for removing diseased fruits or leaves

The storage unit 303 stores data and programs used by the agricultural assistance apparatus 30. In an aspect, the storage unit 303 may store trained models, and information on the user managing the cultivated field.

The control unit 304 controls the operations of the agricultural assistance apparatus 30 by reading programs stored in the storage unit 303 and executing instructions in the programs. The control unit 304 may be an application processor, for example. The control unit 304 operates according to the programs to serve as an image obtainment unit 3031, a model obtainment unit 3032, a parameter estimation unit 3033, and an operation instruction unit 3034.

The image obtainment unit 3031 controls the operation of obtaining images or videos captured by the imaging mechanism 350 to be described later. The images or videos obtained by the image obtainment unit 3031 may be in any format. For example, the image obtainment unit 3031 may obtain images or videos in formats including the following.
- JPEG (Joint Photographic Experts Group)
- PNG (Portable Network Graphics)
- GIF (Graphics Interchange Format)
- TIFF (Tagged Image File Format)
- bitmap
- MP4 (Moving Picture Experts Group)
- MOV (QuickTime file format)
- AVI (Audio Video still Images)
- VOB (Video Object file)

The model obtainment unit 3032 controls the processing of obtaining trained models from the server 20. Specifically, for example, the model obtainment unit 3032 obtains a trained model in response to the image obtainment unit 3031 obtaining an image of a plant. The model obtainment unit 3032 may save the obtained trained model in the storage unit 303 so that repeated obtainment of the trained model can be avoided on future occasions of performing operations for the same plant. Alternatively, the model obtainment unit 3032 may obtain a trained model and temporarily store it in nonvolatile memory on every occasion of performing an operation for the plant.

The latter manner allows the user to perform trained model-based operations using an agricultural assistance apparatus with a limited storage capacity.

The parameter estimation unit 3033 controls the processing of estimating the values of predetermined parameters for predetermined sites in plants based on plant images obtained by the image obtainment unit 3031, and trained models obtained by the model obtainment unit. Specifically, the predetermined sites may include the following, for example.
- a flower and petals of the plant
- a pistil of the plant
- stamens of the plant
- a leaf of the plant
- a fruit of the plant
- a stem of the plant

The predetermined parameters may include the following, for example.
- the orientation of the above predetermined sites in the plant
- the positional relationship of the above predetermined sites in the plant

As values of the orientation parameter, the parameter estimation unit 3033 may calculate normal vectors in a three-dimensional space, rather than values such as upward and downward. For example, the normal vectors in the three-dimensional space may be vectors that indicate directions in a 360° spherical model.

Thus, for flowers or leaves of a plant oriented in various directions, the user managing the cultivated field can accurately estimate the directions and appropriately perform an operation such as pollination, harvesting, or removing leaves.

The operation instruction unit 3034 controls the processing of causing the operation mechanism 302 of the agricultural assistance apparatus 30 to perform predetermined operations. The predetermined operations may include operations for the following tasks, for example.
- pollination (applying pollen to pistils)
- harvesting
- removing leaves (removing overlapping leaves and excess leaves)
- spreading fertilizer
- thinning out for removing diseased fruits or leaves

The imaging mechanism 350 captures images of plants in the cultivated field. The imaging mechanism 350 may also capture videos of the plants.

The imaging mechanism 350 may be any existing camera without limitation. The imaging mechanism 350 may be a silver-halide film camera or may be a digital camera.

In an aspect, depending on its use, the agricultural assistance apparatus 30 may have functions other than those listed above. For example, the agricultural assistance apparatus may have functions including the following (not shown).
- a water sprinkler for sprinkling water
- a fertilizer spreader for spreading fertilizer
- chemicals spreader for spreading chemicals

The additional functions are not limited to the above and may include any function necessary for agriculture.

### <1.3 Structure of agricultural assistance apparatus 30>

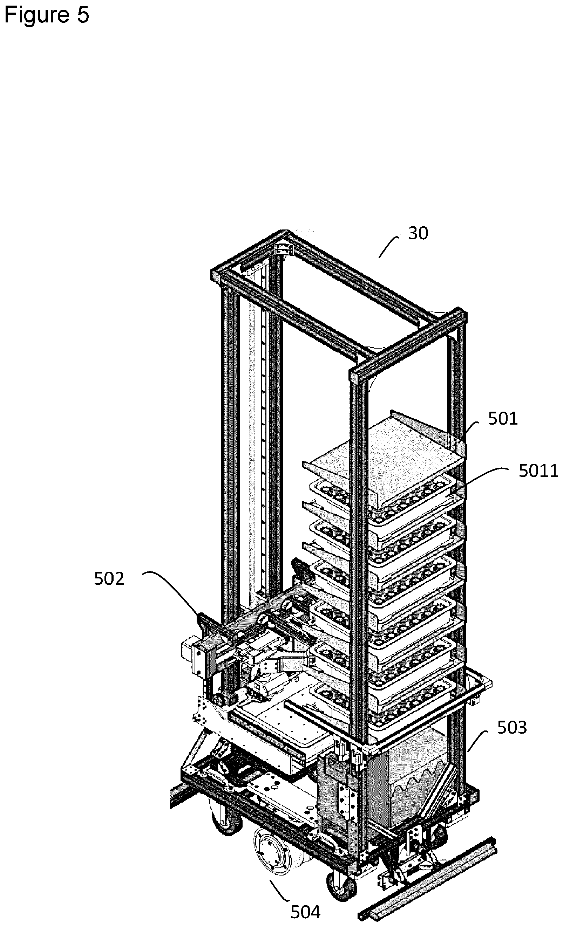

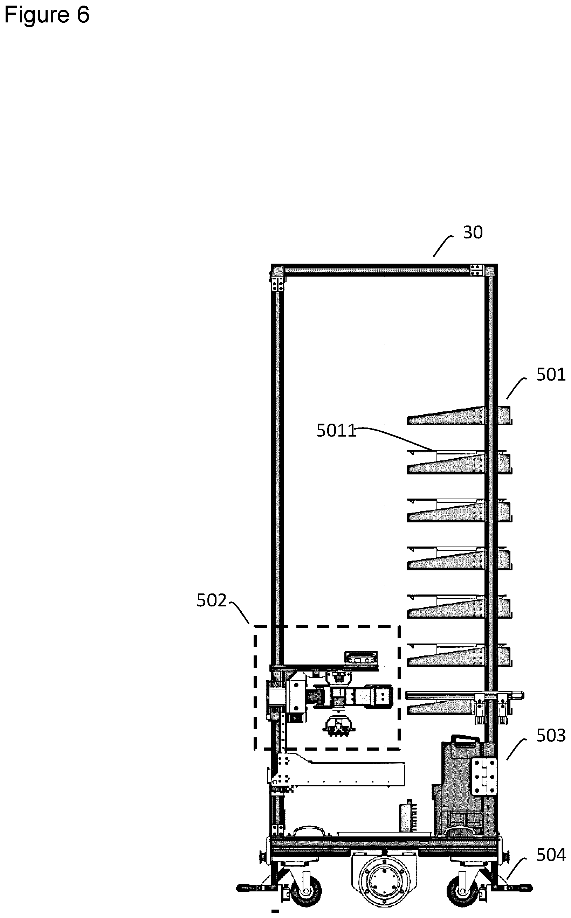

Figures 5 and 6 are diagrams illustrating exemplary external views of the agricultural assistance apparatus 30 according to the first embodiment. Figure 5 is a perspective view of the agricultural assistance apparatus 30, and Figure 6 is a side view of the agricultural assistance apparatus 30.

As illustrated in Figures 5 and 6, the agricultural assistance apparatus 30 includes a tray rack 501, harvest trays 5011, a main unit 502, an information processing unit 503, and a running unit 504. The main unit 502 may be disposed on rails that allow vertical movements.

The tray rack 501 and the harvest trays 5011 are a mechanism for holding crops, such as fruits, harvested by the agricultural assistance apparatus 30. Specifically, for example, the harvest trays 5011 for holding harvested fruits are mounted on the tray rack 501 of the agricultural assistance apparatus 30. The agricultural assistance apparatus 30 puts harvested fruits (e.g., strawberries) on the harvest trays 5011. The tray rack 501 is multitiered; when one harvest tray 5011 reaches its predetermined limit of the amount of content therein, the main unit 502 moves vertically to put fruits on another available harvest tray 5011.

Thus, the user managing the cultivated field can efficiently harvest fruits.

In an aspect, the harvest trays 5011 may be mounted on the tray rack 501 by the user managing the cultivated field, or even automatically. Specifically, the main unit 502 of the agricultural assistance apparatus 30 may have a tray mounting arm (not shown) movable backward and forward and rightward and leftward. The tray mounting arm may grab and move a harvest tray 5011 to set it at a predetermined place in the tray rack 501.

Thus, if the number of tiers of the tray rack of the agricultural assistance apparatus 30 increases, the user managing the cultivated field can still eliminate the need to manually mount the trays.

In an aspect, the tray rack 501 and the harvest trays 5011 may hold seedlings rather than harvested fruits. In this case, the main unit 502 may perform the operation of successively taking the seedlings out of the harvest trays 5011 and planting them in the cultivated field.

The main unit 502 is a mechanism for performing tasks in the cultivated field (such as pollination, harvesting, and removing leaves), as will be described in detail later.

The information processing unit 503 is a mechanism for the agricultural assistance apparatus 30 to communicate with external terminals, or to provide operation instructions to components such as the main unit 502 based on programs stored in the information processing unit 503. The information processing unit 503 may be a computer having a processor or may be a microcomputer.

The running unit 504 is a mechanism for moving the agricultural assistance apparatus 30 within the cultivated field. Specifically, for example, the running unit 504, which is provided on the bottom surface of the agricultural assistance apparatus 30, may be wheels for running on the ground or wheels movable on rails. The running unit 504 may also take the following forms to move the agricultural assistance apparatus 30.
- a mechanism that includes a magnet sensor and runs the agricultural assistance apparatus 30 by detecting magnets installed on the surface of paths in the cultivated field
- a mechanism that includes a line sensor and runs the agricultural assistance apparatus 30 by detecting lines drawn on the cultivated field

Further, the running unit 504 may have any mechanism used for self-driving of vehicles.

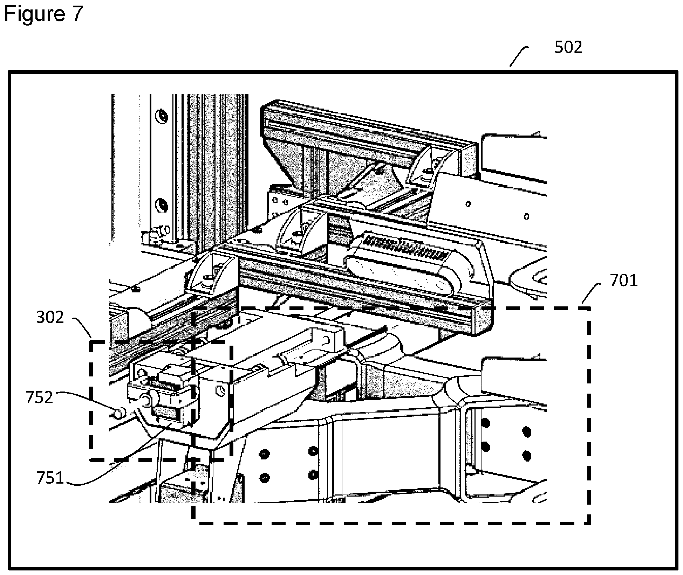

Figure 7 is a diagram illustrating a detailed structure of the main unit 502 of the agricultural assistance apparatus 30.

As illustrated in Figure 7, the agricultural assistance apparatus 30 includes: the main unit 502; an arm 701 disposed on the main unit 502; and the operation mechanism 302, a driven mechanism 751, and an attachment 752 provided at the tip of the arm.

The operation mechanism 302 may be provided at the tip of the arm 701. The imaging mechanism 350 (not shown) may be provided in the operation mechanism 302 (i.e., at the tip of the arm 701) or at any other position on the arm 701.

In an aspect, the arm 701 may include a pollination imaging unit (not shown) that captures an image of the moment of pollination, and a yield prediction unit (not shown) that predicts a yield based on the captured image of the moment of pollination. In addition, the agricultural assistance apparatus 30 may present the predicted yield to the user. Thus, the user managing the cultivated field can determine, based on the information on the moment of pollination and on the predicted yield, whether pollination was appropriately performed.

In an aspect, if the predicted yield is lower than past predicted yields, the agricultural assistance apparatus 30 may perform the pollination again.

Thus, the user managing the cultivated field can avoid insufficient pollination to stably harvest crops such as fruits.

As illustrated in Figure 7, the operation mechanism 302 is provided at the tip of the movable arm 701 to perform predetermined operations while in contact with flowers, fruits, or leaves of plants. The operation mechanism 302 may adopt different mechanisms at its tip for different operation types. For example, for the operation of pollinating plants, the tip to be in contact with flowers of plants may have a fibrous member. For the operation of harvesting fruits or removing leaves, the tip may have an arm for harvesting fruits or pruning. The operation mechanism 302 uses various such mechanisms at its tip to perform predetermined operations for plants. The following describes actions in performing pollination.

For example, the operation mechanism 302 may include the driven mechanism 751 and the attachment 752. According to control by the control unit 304, the operation mechanism 302 drives the driven mechanism 751 to bring the attachment 752 into contact with a predetermined site (e.g., the pistil of a flower) of a plant to be pollinated. That is, based on an image obtained from the imaging mechanism 350 and a trained model, the control unit 304 outputs the position of the pistil in the image, and a normal vector for the pistil. According to the information on the normal vector output by the control unit 304, the operation mechanism 302 drives the driven mechanism 751 in three-dimensional directions to control the attachment 752 to contact the pistil perpendicularly (i.e., exactly from front of the pistil) to thoroughly and uniformly apply pollen to the pistil. The operation mechanism 302 may be moved crisscross while the attachment 752 is in contact with the pistil and stamens of the flower.

### <2 Operations>

Now, a process will be described in which the agricultural assistance apparatus 30 in the system 1 performs a predetermined operation based on a plant image and a trained model obtained.

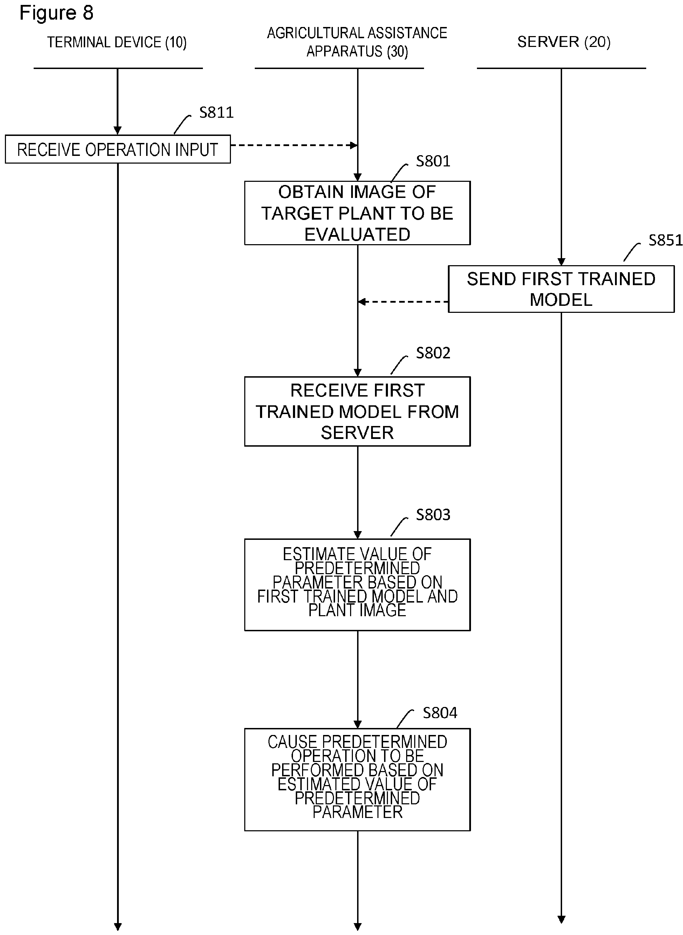

Figure 8 is a flowchart illustrating a process in which the agricultural assistance apparatus 30 performs a predetermined operation based on a plant image and a trained model obtained.

At step S811, the control unit 180 of the terminal device 10 receives operation input from the user managing the cultivated field. Specifically, for example, the control unit 180 receives, from the user managing the cultivated field, operation input for a predetermined operation (a task such as pollination or harvesting fruits) in the cultivated field. At this point, the control unit 180 may send, to the agricultural assistance apparatus 30, the details of the operation that are input by the user, or information on the operation that is preset for the user managing the cultivated field.

At step S801, the control unit 304 of the agricultural assistance apparatus 30 obtains an image of a target plant to be evaluated. Specifically, for example, the agricultural assistance apparatus 30 moves along rails under the apparatus to front of the target plant and captures an image of the plant with the imaging mechanism 350. The control unit 304 may also send the captured image to the server 20. Specifically, the image may be captured by, for example, the imaging mechanism 350 automatically identifying a site such as a flower and capturing an image focused on the site such as a flower. Further, the distance and angle between the operation mechanism 302 and the captured site such as a flower may be calculated, for example using a sensor such as an infrared sensor or a depth sensor that detects the distance and angle.

At step S851, the control unit 203 of the server 20 sends the first trained model to the agricultural assistance apparatus 30. Specifically, for example, the control unit 203 may send the first trained model trained in the above-described manner to the agricultural assistance apparatus 30 according to an instruction from the agricultural assistance apparatus 30. The trained model to be sent is not limited to the first trained model. The control unit 203 may determine, based on the plant image obtained by the agricultural assistance apparatus 30, the trained model to be sent. Alternatively, the control unit 203 may receive information on the trained model to be sent from the user managing the cultivated field.

At step S802, the control unit 304 of the agricultural assistance apparatus 30 receives the first trained model from the server. Specifically, the model obtainment unit 3032 in the control unit 304 obtains one of the trained models in response to the image obtainment unit 3031 obtaining the plant image. The model obtainment unit 3032 may save the obtained trained model in the storage unit 303 so that repeated obtainment of the trained model can be avoided on future occasions of performing operations for the same plant. Alternatively, the model obtainment unit 3032 may obtain a trained model and temporarily store it in nonvolatile memory on every occasion of performing an operation for the plant.

At step S803, based on the first trained model and the plant image, the control unit 304 of the agricultural assistance apparatus 30 estimates the value of a predetermined parameter. Specifically, based on the plant image obtained by the image obtainment unit 3031 and the trained model obtained by the model obtainment unit, the parameter estimation unit 3033 in the control unit 304 controls the processing of estimating the value of the predetermined parameter for a predetermined site in the plant. Specifically, predetermined sites may include the following, for example.
- a flower and petals of the plant
- a pistil of the plant
- stamens of the plant
- a leaf of the plant
- a fruit of the plant
- a stem of the plant

Predetermined parameters may include the following, for example.
- the orientation of the above predetermined sites in the plant
- the positional relationship of the above predetermined sites in the plant

As values of the orientation parameter, the parameter estimation unit 3033 may calculate normal vectors in a three-dimensional space, rather than values such as upward and downward. For example, the normal vectors in the three-dimensional space may be vectors that indicate directions in a 360° spherical model.

At step S804, the control unit 304 of the agricultural assistance apparatus 30 causes a predetermined operation to be performed based on the estimated value of the predetermined parameter. Specifically, the operation instruction unit 3034 in the control unit 304 controls the processing of causing the operation mechanism 302 of the agricultural assistance apparatus 30 to perform the predetermined operation. Predetermined operations may include operations for the following tasks, for example.
- pollination (applying pollen to pistils)
- harvesting
- removing leaves (removing overlapping leaves and excess leaves)
- thinning out fruits
- spreading fertilizer
- thinning out for removing diseased fruits or leaves

Through the above processing, the user managing the cultivated field can appropriately perform various sorts of processing (such as pollination, harvesting, and removing leaves) for sites in plants (such as flowers, pistils and stamens, leaves, stems, and fruits) that are in any orientation or any positional relationship. This enables stably growing higher quality fruits.

In an aspect, the server 20 may store a result regarding fruit quality when cross-pollinating a self-pollinating plant species. If cross-pollinating the self-pollinating plant species is expected to improve the fruit quality in spite of the self-pollinating plant species based on the result, the server 20 may notify the user managing the cultivated field of that fact.

Thus, the user managing the cultivated field can appropriately manage operations such as pollination, including quality improvement.

### <3 Exemplary operations>

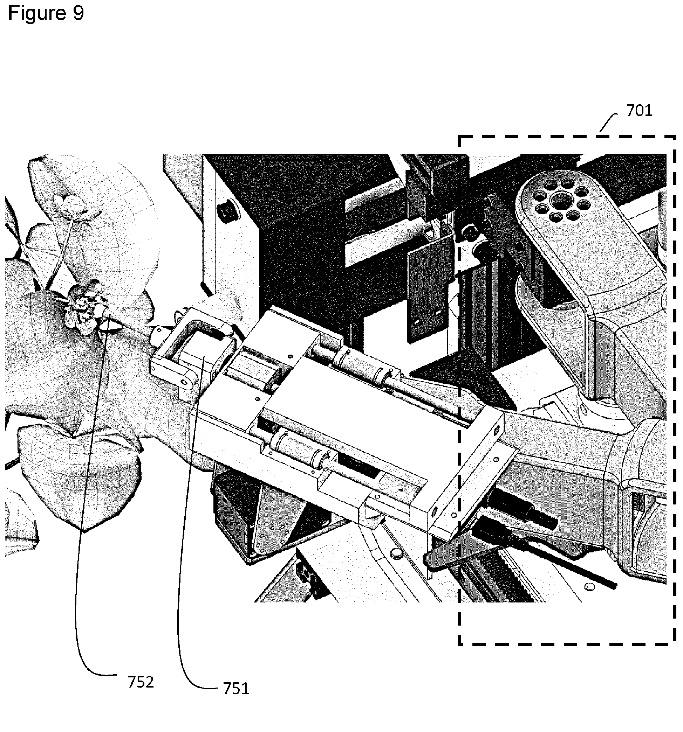

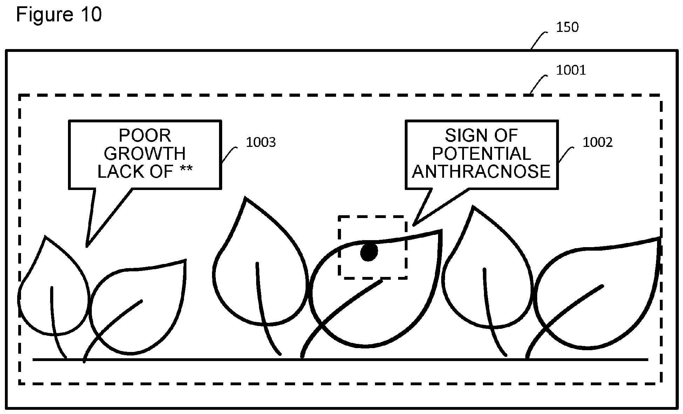

Figures 9 and 10 are diagrams illustrating examples, such as actions in a process in which the system 1 disclosed in the present invention operates components such as the operation mechanism of the agricultural assistance apparatus based on input, received from the user managing the cultivated field, for performing a predetermined operation in the cultivated field.

Figure 9 is a diagram illustrating actions through which the operation mechanism 302 of the agricultural assistance apparatus 30 performs a predetermined operation for a predetermined site in a plant based on the value of a predetermined parameter.

In Figure 9, the agricultural assistance apparatus 30 drives the arm 701 based on the value of the predetermined parameter for the predetermined site in the plant, as identified from a plant image and a trained model. Figure 9 illustrates exemplary actions in pollinating the pistil of a flower. As shown in Figure 9, based on the parameter value identified, the agricultural assistance apparatus 30 moves the arm 701, as well as the driven mechanism 751 of the operation mechanism 302, in predetermined directions. Thus, the attachment 752 of the operation mechanism 302 at the tip of the arm 701 comes perpendicular to (exactly in front of) the pistil of the flower. For example, the predetermined directions, which may be output from the trained model, may be a normal vector determined to be a combination of the following vectors.
- roll (rotation about the x-axis)
- pitch (rotation about the y-axis)
- yaw (rotation about the z-axis)

In this manner, from information on the plant image (two-dimensional information), the direction in the three-dimensional space can be output based on the trained model. By combining the movements of the arm 701 and the movements of the driven mechanism 751, the agricultural assistance apparatus 30 can direct the attachment 752 in any direction to bring the attachment 752 into contact with the plant. Thus, the user managing the cultivated field can appropriately perform an operation, such as pollination, for sites such as flower pistils oriented along the depth with respect to the agricultural assistance apparatus 30.

The above-described operation is not limited to pollination. Other operations occurring in a cultivated field (such as removing leaves, pruning, and thinning out fruits) may be performed in similar manners.

Figure 10 illustrates an exemplary screen that displays notifications to the user managing the cultivated field when tendencies to unhealthiness of plants are detected by the operation mechanism 302 of the agricultural assistance apparatus 30.

In Figure 9, the display 150 of the terminal device 10 carried by the user managing the cultivated field shows a captured image 1001, a disease alert 1002, and a poor-growth alert 1003. The captured image 1001 is a plant image captured by the imaging mechanism 350 included in the agricultural assistance apparatus 30.

The disease alert 1002 is an alert displayed for a plant having a tendency to a disease in the plant image captured. Specifically, the control unit 304 of the agricultural assistance apparatus 30 estimates, based on the plant image obtained and the above-described fourth trained model, the possibility that a tendency seen in the plant is attributed to a disease. If the plant is estimated to have a disease, the disease alert 1002 is presented to the user managing the cultivated field. In an aspect, if the plant has a disease, the agricultural assistance apparatus 30 may also cause the operation mechanism 302 to remove the diseased part, in addition to presenting the disease alert 1002 to the user managing the cultivated field.

Thus, the user managing the cultivated field can discover a disease in its early stage to prevent damages that would be caused by the disease.

The poor-growth alert 1003 is an alert displayed for a plant that shows a tendency to poor growth in the plant image captured. Specifically, the control unit 304 of the agricultural assistance apparatus 30 estimates, based on the plant image obtained and the above-described fifth trained model, the possibility that a tendency seen in the plant is attributed to poor growth. If the plant is estimated to be poorly growing, the poor-growth alert 1003 is presented to the user managing the cultivated field. In an aspect, if the plant is poorly growing, the agricultural assistance apparatus 30 may also cause a fertilizer adjustment unit to adjust fertilizer for the area in the cultivated field where the poorly growing plant is grown, in addition to presenting the poor-growth alert 1003 to the user managing the cultivated field.

Thus, the user managing the cultivated field can discover, in an early stage, poor growth due to causes such as unevenly spread fertilizer. This contributes to harvesting fruits of uniform quality.

In an aspect, the agricultural assistance apparatus may also provide, to the user managing the cultivated field, a similar notification based on the result of determining the overlapping state of leaves.

The embodiment in the present disclosure has illustrated the agricultural assistance system in which the server 20 and the agricultural assistance apparatus 30 communicate information with each other. However, other embodiments are also possible. For example, the above process may be performed without communication with the server 20 but based on trained models stored in the storage unit of the agricultural assistance apparatus 30. Further, the control unit of the agricultural assistance apparatus 30 may have a function of generating three-dimensional models and trained models.

### <4 Variations>

Variations of the embodiment will be described. As variations, the following aspects may be adopted.
(1) An information processing apparatus having a relevant program, which may be preinstalled or post-installed in the apparatus, or may be stored in an external non-transitory storage medium or may be run by cloud computing.
(2) A method for causing a computer to function as an information processing apparatus having a relevant program, which may be preinstalled or post-installed in the apparatus, or may be stored in an external non-transitory storage medium or may be run by cloud computing.

### <Supplement>

The following is a supplement to the above description of the embodiment.

### (Supplementary item 1)

An agricultural assistance system 1 including: an operation mechanism 302 that contacts a plant to perform a predetermined operation; and a control unit 304, the control unit 304 including: a model generation unit 2033 that generates a three-dimensional model of a plant based on a combination of predetermined generation conditions; a training unit 2034 that generates a first trained model trained with training data, the training data including: a value of a predetermined parameter in the three-dimensional model; and an image of a plant; an image obtainment unit 3041 that obtains an image of a target plant to be evaluated; a model obtainment unit 3042 that obtains the first trained model; a parameter estimation unit 3043 that estimates, based on the first trained model and the image of the target plant to be evaluated, a value of the predetermined parameter for a predetermined site in the target plant to be evaluated; and an operation instruction unit 3044 that causes the operation mechanism 302 to perform the predetermined operation based on the estimated value of the predetermined parameter.

### (Supplementary item 2)

The agricultural assistance system 1 according to supplementary item 1, wherein the predetermined parameter is at least one selected from the group consisting of an orientation of the predetermined site in the plant and a positional relationship of the predetermined site in the plant, in a three-dimensional space.

### (Supplementary item 3)

The agricultural assistance system 1 according to supplementary item 1 or 2, wherein the predetermined generation conditions include at least one selected from the group consisting of a size of the three-dimensional model, a number of petals in the three-dimensional model, a color of the three-dimensional model, a shape of the three-dimensional model, a background in a space in which the three-dimensional model is generated, a position of illumination in the space in which the three-dimensional model is generated, and an orientation of the three-dimensional model.

### (Supplementary item 4)

The agricultural assistance system 1 according to any of supplementary items 1 to 3, wherein the predetermined site in the target plant to be evaluated is at least one position selected from the group consisting of a flower pistil, a flower stamen, a fruit, and a leaf.

### (Supplementary item 5)

The agricultural assistance system 1 according to any of supplementary items 1 to 4, wherein the predetermined operation is at least one operation selected from the group consisting of pollinating flowers, harvesting fruits, removing leaves, and thinning out fruits.

### (Supplementary item 6)

The agricultural assistance system 1 according to any of supplementary items 1 to 5, including an imaging mechanism 350 that captures images of plants.

### (Supplementary item 7)

The agricultural assistance system 1 according to any of supplementary items 1 to 6, wherein the model obtainment unit 3032 obtains a second trained model generated by training with: at least one of ventilation and sunlight in a cultivated field; and a fruit yield in a harvest season.

### (Supplementary item 8)

The agricultural assistance system 1 according to supplementary item 7, wherein the control unit 304 includes a positional relationship estimation unit that estimates, based on a predicted yield that is output from the second trained model, an optimal positional relationship of any one selected from the group consisting of leaves, flowers, and fruits, and the operation instruction unit 3044 causes the operation mechanism 302 to perform the predetermined operation based on the optimal positional relationship of any one selected from the group consisting of leaves, flowers, and fruits, estimated by the positional relationship estimation unit.

### (Supplementary item 9)

The agricultural assistance system 1 according to any of supplementary items 6 to 8, wherein the control unit 304 includes: a pollination imaging unit that causes the imaging mechanism 350 to capture an image of a moment of pollination; a yield prediction unit that predicts a yield based on the image of the moment of pollination captured; and an output unit that presents the predicted yield to a user.

### (Supplementary item 10)

The agricultural assistance system 1 according to any of supplementary items 1 to 9, further including: a weight sensor that measures weights of fruits; and a sugar content sensor that measures sugar contents of fruits, wherein the model obtainment unit 3032 obtains a third trained model that associates an image of a fruit, a weight of the fruit, a sugar content of the fruit, and quality of the fruit with each other, and the output unit determines quality of a fruit based on an image, a weight, and a sugar content of the fruit and on the third trained model, and outputs the quality of the fruit.

### (Supplementary item 11)

The agricultural assistance system 1 according to any of supplementary items 1 or 10, wherein the model obtainment unit 3032 obtains a fourth trained model that associates an image of a plant with an image of the plant having a disease, the output unit provides, in response to receiving input of an image of a plant and based on the fourth trained model, output indicating whether the plant has a disease, and, if the output indicates that the plant has a disease, the operation instruction unit 3044 causes the operation mechanism 302 to perform an operation of removing a site affected with the disease.

### (Supplementary item 12)

The agricultural assistance system 1 according to any of supplementary items 1 to 11, wherein the control unit 304 includes a fertilizer adjustment unit, the model obtainment unit 3032 obtains a fifth trained model that associates an image of a plant with a nutrition state of the plant, the output unit determines, in response to receiving input of an image of a plant and based on the fifth trained model, overnutrition or undernutrition of the plant and provides output indicating a nutrition state of the plant, and the fertilizer adjustment unit adjusts, based on the nutrition state of the plant, fertilizer to be spread in a cultivated field.

### (Supplementary item 13)

An agricultural assistance apparatus 30 including: an operation mechanism 302 that contacts a plant to perform a predetermined operation; and a control unit 304, the control unit 304 including: a model generation unit 2033 that generates a three-dimensional model of a plant based on a combination of predetermined generation conditions; a training unit 2034 that generates a first trained model trained with training data, the training data including: a value of a predetermined parameter in the three-dimensional model; and an image of a plant; an image obtainment unit 3041 that obtains an image of a target plant to be evaluated; a model obtainment unit 3042 that obtains the first trained model; a parameter estimation unit 3043 that estimates, based on the first trained model and the image of the target plant to be evaluated, a value of the predetermined parameter for a predetermined site in the target plant to be evaluated; and an operation instruction unit 3044 that causes the operation mechanism 302 to perform the predetermined operation based on the estimated value of the predetermined parameter.

### (Supplementary item 14)

An agricultural assistance method using: an operation mechanism 302 that contacts a plant to perform a predetermined operation; and a control unit 304, the method including performing, by the control unit 304: a model generation step of generating a three-dimensional model of a plant based on a combination of predetermined generation conditions; a training step of generating a first trained model trained with training data, the training data including: a value of a predetermined parameter in the three-dimensional model; and an image of a plant; an image obtainment step (S801) of obtaining an image of a target plant to be evaluated; a model obtainment step (S802) of obtaining the first trained model; a parameter estimation step (S803) of estimating, based on the first trained model and the image of the target plant to be evaluated, a value of the predetermined parameter for a predetermined site in the target plant to be evaluated; and an operation instruction step (S804) of causing the operation mechanism 302 to perform the predetermined operation based on the estimated value of the predetermined parameter.

### (Supplementary item 15)

A program for causing a computer 20 to perform agricultural assistance using: an operation mechanism 302 that contacts a plant to perform a predetermined operation; and a control unit 304, the program causing the control unit 304 to perform: a model generation step of generating a three-dimensional model of a plant based on a combination of predetermined generation conditions; a training step of generating a first trained model trained with training data, the training data including: a value of a predetermined parameter in the three-dimensional model; and an image of a plant; an image obtainment step (S801) of obtaining an image of a target plant to be evaluated; a model obtainment step (S802) of obtaining the first trained model; a parameter estimation step (S803) of estimating, based on the first trained model and the image of the target plant to be evaluated, a value of the predetermined parameter for a predetermined site in the target plant to be evaluated; and an operation instruction step (S804) of causing the operation mechanism 302 to perform the predetermined operation based on the estimated value of the predetermined parameter.

### [REFERENCE SIGNS LIST]

- 1: system
- 10: terminal device
- 12: communication interface
- 13: input device
- 14: output device
- 15: memory
- 16: storage unit
- 19: processor
- 20: server
- 22: communication interface
- 23: input/output interface
- 25: memory
- 26: storage
- 29: processor
- 30: agricultural assistance apparatus
- 32: communication interface
- 33: input/output interface
- 35: memory
- 36: storage unit
- 39: processor
- 80: network
- 170: storage unit
- 180: control unit
- 1801: input operation reception unit
- 1802: transmission/reception unit
- 1803: data processing unit
- 1804: notification control unit
- 130: operation reception section
- 1301: keyboard
- 1302: mouse
- 140: audio processing unit
- 141: microphone
- 142: speaker
- 150: display
- 160: location information sensor
- 202: storage unit
- 2021: trained model database
- 203: control unit
- 2031: reception control unit
- 2032: transmission control unit
- 2033: model generation unit
- 3034: training unit
- 302: operation mechanism
- 303: storage unit
- 304: control unit
- 3041: image obtainment unit
- 3042: model obtainment unit
- 3043: parameter estimation unit
- 3044: operation instruction unit
- 350: imaging mechanism

## Claims

1. An agricultural assistance system comprising:
an operation mechanism that contacts a plant to perform a predetermined operation; and
a control unit,
the control unit comprising:
a model generation unit that generates a three-dimensional model of a plant based on a combination of predetermined generation conditions;
a training unit that generates a first trained model trained with training data, the training data comprising: a value of a predetermined parameter in the three-dimensional model; and an image of a plant;
an image obtainment unit that obtains an image of a target plant to be evaluated;
a model obtainment unit that obtains the first trained model;
a parameter estimation unit that estimates, based on the first trained model and the image of the target plant to be evaluated, a value of the predetermined parameter for the predetermined site in the target plant to be evaluated; and
an operation instruction unit that causes the operation mechanism to perform the predetermined operation based on the estimated value of the predetermined parameter.

2. The agricultural assistance system according to claim 1, wherein the predetermined parameter is at least one selected from the group consisting of an orientation of the predetermined site in the plant and a positional relationship of the predetermined site in the plant, in a three-dimensional space for generating the three-dimensional model.

3. The agricultural assistance system according to claim 1 or 2, wherein the predetermined generation conditions comprise at least one selected from the group consisting of a size of the three-dimensional model, a number of petals in the three-dimensional model, a color of the three-dimensional model, a shape of the three-dimensional model, a background in a space in which the three-dimensional model is generated, a position of illumination in the space in which the three-dimensional model is generated, and an orientation of the three-dimensional model.

4. The agricultural assistance system according to any of claims 1 to 3, wherein the predetermined site in the target plant to be evaluated is at least one position selected from the group consisting of a flower pistil, a flower stamen, a fruit, and a leaf.

5. The agricultural assistance system according to any of claims 1 to 4, wherein the predetermined operation is at least one operation selected from the group consisting of pollinating flowers, harvesting fruits, removing leaves, and thinning out fruits.

6. The agricultural assistance system according to any of claims 1 to 5, comprising an imaging mechanism that captures images of plants.

7. The agricultural assistance system according to any of claims 1 to 6, wherein the model obtainment unit obtains a second trained model generated by training with: at least one of ventilation and sunlight in a cultivated field; and a fruit yield in a harvest season.

8. The agricultural assistance system according to claim 7, wherein
the control unit comprises a positional relationship estimation unit that estimates, based on a predicted yield that is output from the second trained model, an optimal positional relationship of any one selected from the group consisting of leaves, flowers, and fruits, and
the operation instruction unit causes the operation mechanism to perform the predetermined operation based on the optimal positional relationship of any one selected from the group consisting of leaves, flowers, and fruits, estimated by the positional relationship estimation unit.

9. The agricultural assistance system according to claim 6, wherein the control unit comprises:
a pollination imaging unit that causes the imaging mechanism to capture an image of a moment of pollination;
a yield prediction unit that predicts a yield based on the image of the moment of pollination captured; and
an output unit that presents the predicted yield to a user.

10. The agricultural assistance system according to claim 9, further comprising:
a weight sensor that measures weights of fruits; and
a sugar content sensor that measures sugar contents of fruits,
wherein
the model obtainment unit obtains a third trained model that associates an image of a fruit, a weight of the fruit, a sugar content of the fruit, and quality of the fruit with each other, and
the output unit determines quality of a fruit based on an image, a weight, and a sugar content of the fruit and on the third trained model, and outputs the quality of the fruit.

11. The agricultural assistance system according to claim 9 or 10, wherein
the model obtainment unit obtains a fourth trained model that associates an image of a plant with an image of the plant having a disease,
the output unit provides, in response to receiving input of an image of a plant and based on the fourth trained model, output indicating whether the plant has a disease, and
if the output indicates that the plant has a disease, the operation instruction unit causes the operation mechanism to perform an operation of removing a site affected with the disease.

12. The agricultural assistance system according to any of claims 9 to 11, wherein
the control unit comprises a fertilizer adjustment unit,
the model obtainment unit obtains a fifth trained model that associates an image of a plant with a nutrition state of the plant,
the output unit determines, in response to receiving input of an image of a plant and based on the fifth trained model, overnutrition or undernutrition of the plant and provides output indicating a nutrition state of the plant, and
the fertilizer adjustment unit adjusts, based on the nutrition state of the plant, fertilizer to be spread in a cultivated field.

13. An agricultural assistance apparatus comprising:
an operation mechanism that contacts a plant to perform a predetermined operation; and
a control unit,
the control unit comprising:
a model generation unit that generates a three-dimensional model of a plant based on a combination of predetermined generation conditions;
a training unit that generates a first trained model trained with training data, the training data comprising: a value of a predetermined parameter in the three-dimensional model; and an image of a plant;
an image obtainment unit that obtains an image of a target plant to be evaluated;
a model obtainment unit that obtains the first trained model;
a parameter estimation unit that estimates, based on the first trained model and the image of the target plant to be evaluated, a value of the predetermined parameter for the predetermined site in the target plant to be evaluated; and
an operation instruction unit that causes the operation mechanism to perform the predetermined operation based on the estimated value of the predetermined parameter.

14. An agricultural assistance method using:
an operation mechanism that contacts a plant to perform a predetermined operation; and
a control unit,
the method comprising performing, by the control unit:
a model generation step of generating a three-dimensional model of a plant based on a combination of predetermined generation conditions;
a training step of generating a first trained model trained with training data, the training data comprising: a value of a predetermined parameter in the three-dimensional model; and an image of a plant;
an image obtainment step of obtaining an image of a target plant to be evaluated;
a model obtainment step of obtaining the first trained model;
a parameter estimation step of estimating, based on the first trained model and the image of the target plant to be evaluated, a value of the predetermined parameter for the predetermined site in the target plant to be evaluated; and
an operation instruction step of causing the operation mechanism to perform the predetermined operation based on the estimated value of the predetermined parameter.

15. A program for causing a computer to perform agricultural assistance using:
an operation mechanism that contacts a plant to perform a predetermined operation; and
a control unit,
the program causing the control unit to perform:
a model generation step of generating a three-dimensional model of a plant based on a combination of predetermined generation conditions;
a training step of generating a first trained model trained with training data, the training data comprising: a value of a predetermined parameter in the three-dimensional model; and an image of a plant;
an image obtainment step of obtaining an image of a target plant to be evaluated;
a model obtainment step of obtaining the first trained model;
a parameter estimation step of estimating, based on the first trained model and the image of the target plant to be evaluated, a value of the predetermined parameter for the predetermined site in the target plant to be evaluated; and
an operation instruction step of causing the operation mechanism to perform the predetermined operation based on the estimated value of the predetermined parameter.
